(19) **Europäisches Patentamt / European Patent Office / Office européen des brevets**

(11) **EP 3 031 453 B1**

(12) **EUROPEAN PATENT SPECIFICATION**

(45) Date of publication and mention
of the grant of the patent:
**28.02.2018 Bulletin 2018/09**

(51) Int Cl.:
*A61K 9/48* (2006.01)          *A61K 9/54* (2006.01)
*A61K 47/30* (2006.01)

(21) Application number: **14834946.7**

(22) Date of filing: **17.01.2014**

(86) International application number:
**PCT/KR2014/000510**

(87) International publication number:
**WO 2015/020286 (12.02.2015 Gazette 2015/06)**

(54) **STABLE TRIPLE-LAYER CAPSULE USING POORLY WATER-SOLUBLE SUBSTANCE, MANUFACTURING METHOD THEREFOR AND COSMETIC COMPOSITION USING SAME**

STABILE DREILAGIGE KAPSEL MIT EINER SCHLECHT WASSERLÖSLICHEN SUBSTANZ, HERSTELLUNGSVERFAHREN DAFÜR UND KOSMETISCHE ZUSAMMENSETZUNG DAMIT

CAPSULE TRIPLE COUCHE STABLE UTILISANT UNE SUBSTANCE FAIBLEMENT SOLUBLE DANS L'EAU, SON PROCÉDÉ DE FABRICATION ET COMPOSITION COSMÉTIQUE L'UTILISANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.08.2013 KR 20130094275**

(43) Date of publication of application:
**15.06.2016 Bulletin 2016/24**

(73) Proprietor: **Biogenics, Inc.
Daejeon 305-510 (KR)**

(72) Inventors:
• **SON, Tae-Hun
Daejeon 305-759 (KR)**
• **KO, Tae-Sung
Sejong 339-014 (KR)**
• **SHIN, Chan-Jae
Daejeon 305-728 (KR)**
• **JEONG, Seong-Yoon
Daejeon 302-852 (KR)**

• **HAN, Young-Hee
Daedeok-gu
Daejeon (KR)**
• **MAENG, Sung-Ho
Asan-si
Chungcheongnam-do 336-732 (KR)**
• **LEE, Dong-sun
Gangdong-gu,
Seoul (KR)**

(74) Representative: **Winter, Brandl, Fürniss, Hübner, Röss, Kaiser, Polte - Partnerschaft mbB
Patent- und Rechtsanwaltskanzlei
Alois-Steinecker-Strasse 22
85354 Freising (DE)**

(56) References cited:
WO-A1-02/051517          JP-A- 2003 535 032
JP-A- 2005 075 826          KR-A- 20050 040 505
KR-A- 20080 059 380          KR-A- 20090 070 161
KR-B1- 101 242 271

**EP 3 031 453 B1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**TECHNICAL FIELD**

The present invention relates to a three-layered capsule stabilized using a hollow silica hydrogel polymer and a hydrophobic polymer for the sake of protecting a water-insoluble substance from the external harmful environments, and a preparation method for the same.

**BACKGROUND OF THE INVENTION**

Water-insoluble substances for use in cosmetics, such as, for example, carotenoids, ceramide liquid crystals, oil-soluble extracts, dyes, etc., are not easy to use in the cosmetics exposed to different environments, for the deterioration in the efficacies, colors and skin feel as caused by the instability, colors or oiliness of the water-insoluble substances tend to worsen with the materials contained in the cosmetic formulations, especially, oxygen, water, oils, surfactants, etc. or the heat generated in the preparation process of the cosmetic formulations. For solving this problem, many approaches including liposome preparation, emulsification, capsulation, etc. have been made, with no substantial solution to the problem.

As for the approaches suggested to overcome the problem, Korean Patent Publication No. 10-2009-0070161 discloses the preparation of a polymer capsule having a double-layered structure that uses cationic and anionic polymers according to the coacervation method to stabilize carotenoids. In addition, Korean Patent Publication No. 10-2009-0122666 describes the preparation of a capsule containing lecithin as an oil-soluble substance according to the low-temperature cooling process.

Capsules prepared using different methods other than the above-mentioned methods have also been suggested, but those conventional methods when applied to the actual cosmetic formulation do not contribute to the stability, color and skin feel of the cosmetics as desired by the customers, showing the limitations of their application to the cosmetics. Accordingly, there is an emerging demand for developing products and techniques with improvement of the problem.

**BRIEF SUMMARY OF THE INVENTION**

It is an object of the present invention to provide a three-layered capsule containing a water-insoluble substance and a preparation method for the same, where the three-layered capsule has a form with acquired unique properties peculiar to the hydrophobic-hydrophilic-hydrophobic three-layered structure and is thus sufficiently applicable to different environments thanks to the three-layered structure.

It is another object of the present invention to provide various cosmetic compositions containing the capsule.

In accordance with the present invention, there is provided a stable three-layered capsule using a water-insoluble substance that includes: a single-layered capsule comprising a water-insoluble substance, a stabilizer, and a hollow silica; a double-layered capsule having a hydrogel polymer blend surrounding the outside of the single-layered capsule; and a three-layered capsule having an appropriately hydrophobic polymer surrounding the outside of the double-layered capsule.

Preferably, the three-layered capsule has an average particle size of 50 $\mu$m to 3,000 $\mu$m.

In accordance with the present invention, there is further provided a method for preparing a stable three-layered capsule using a water-insoluble substance that includes: mixing a water-insoluble substance, a stabilizer, and a hollow silica with a first solvent to achieve a homogeneous inclusion and then performing a drying to prepare a single-layered capsule, where the first solvent is any one selected from the group consisting of methyl alcohol, ethyl alcohol, and isopropyl alcohol; homogeneously dispersing the single-layered capsule in a second solvent selected from water or hydrous ethanol containing a dissolved hydrogel polymer blend and then performing a drying to prepare a double-layered capsule; and homogeneously dispersing the double-layered capsule in a third solvent selected from ethyl alcohol or hydrous ethanol containing a dissolved hydrophobic polymer and then performing a drying to prepare a three-layered capsule; or dispersing a functional pigment having an additional concealing function in a third solvent containing a dissolved hydrophobic polymer, adding the double-layered capsule to achieve a homogeneous dispersion, and then performing a drying to prepare a three-layered capsule.

**EFFECTS OF INVENTION**

The stable three-layered capsule containing a water-insoluble substance according to the present invention enables the use of materials susceptible to physical/chemical changes with their unstable structure in a stable form under different environments and particularly overcomes the problems with such materials easily harmed by the adverse actions of acidification on the properties to have content deterioration, color change, foul odor, etc. and ready to spoil the beauty of cosmetic compositions with their innate colors.

In particular, the innermost first layer contains water-insoluble substances including a stabilizer as encapsulated with a hollow silica excellent in thermal stability and inclusion capability to oil-soluble substances so as to promote thermal characteristics and chemical stability. The second layer contains the hydrogel polymer blend that prevents the flow of water-soluble substances in and out of the capsule to the maximum according to the osmosis principle and blocks oil-soluble substances with the gel phase immobilizing the water-soluble substances. The outermost third layer contains a hydrophobic polymer that forms a support layer and controls to prevent a rapid entry of the water-soluble substances into the capsule.

For this reason, the inherent functional ingredients of the water-insoluble substances can be maintained in the stable state in the three-layered capsule and, when applied to the skin, realize the efficacies like wrinkle improving and UV blocking effects with antioxidant actions.

In addition, the hydrogel polymer blend used as a capsule wall can modify the strong oiliness of the water-insoluble substances and improve the bad skin feel.

The stabilized three-layered capsule containing water-insoluble substances with the above-mentioned advantages can be used to provide cosmetic compositions in different forms.

## BRIEF DESCRIPTION OF THE SEVERAL VIEWS OF THE DRAWING

FIG. 1 is an SEM (Scanning Electronic Microscopy) image of a three-layered capsule (astaxanthin capsule) prepared in Example 1.
FIG. 2 is an SEM image of a double-layered capsule (astaxanthin capsule) prepared in Comparative Example 2.
FIG. 3 is an SEM image of a single-layered capsule (astaxanthin capsule) prepared in Comparative Example 1.
FIG. 4 is an SEM image of a double-layered capsule encapsulated while the three-layered capsule is broken.
FIG. 5 is an image of the final capsules prepared in Examples 1 to 5 from left to right.
FIG. 6 is a graph showing the change in temperature stability (4) over time in the step-by-step preparation of the capsules using astaxanthin.
FIG. 7 is a graph showing the change in temperature stability (25) over time in the step-by-step preparation of the capsules using astaxanthin.
FIG. 8 is a graph showing the change in temperature stability (50) over time in the step-by-step preparation of the capsules using astaxanthin.
FIG. 9 is a graph showing the change in sunlight stability over time in the step-by-step preparation of the capsules using astaxanthin.

## DETAILED DESCRIPTION OF THE INVENTION

Hereinafter, the present invention will be described in further detail with reference to the following examples, which are given for illustrations of the present invention and not intended to limit the scope of the present invention.

The capsule having a three-layered structure stabilizing a water-insoluble substance according to the present invention consists of a single-layered capsule composed of a water-insoluble substance, a stabilizer, and a hollow silica; a double-layered capsule having a coating of a hydrogel polymer blend on the single-layered capsule; and a three-layered capsule containing a hydrophobic polymer alone or in combination with a functional pigment.

The capsule structure designed as a unique three-layered structure according to the present invention is a combination of a double-layered structure having a hydrogel polymer blend and a three-layered structure having a hydrophobic property. The outermost hydrophobic polymer layer of the capsule that comes in direct contact with the environment of cosmetic formulations has a defense mechanism against water to make the water eventually stuck in or repelled by the hydrogel polymer blend layer having a network structure of water and oil partly penetrating into the hydrophobic polymer layer, thereby disabling penetration of the water. Subsequently, the water stuck in the hydrogel polymer layer keeps equilibrium through the osmosis in the capsule.

As for the defense against heat, the heat is dissipated in a lay-by-lay manner through the three-layered structure and finally blocked by the outer wall of the hollow silica that is stable to heat. Particularly, the outermost capsule layer, which consists of a hydrophobic polymer and a functional pigment, can promote the defensive effect against heat.

The three-layered capsule structure is constructed in order to stabilize the above-mentioned water-insoluble substances. For this purpose, it is necessary to minimize the loss of the water-insoluble substances against the physical/chemical environments that occurs in each step of preparation. Accordingly, for minimization of the loss, a stabilizer is used in the preparation of the single-layered capsule to promote the stability until the step prior to the preparation step for the final three-layered capsule, thereby contributing to the stability of the water-insoluble substances.

More specifically, the step-by-step preparation method will be described as follows.

(1) Preparation of Single-Layered Capsule

The single-layered capsule of the present invention is prepared by mixing a water-insoluble substance, a stabilizer, and a hollow silica with a first solvent to achieve a homogeneous inclusion and then performing a drying. In the single-layered capsule thus prepared, the water-insoluble substances including the stabilizer are immobilized or encapsulated by the hollow silica that is excellent in thermal stability and inclusion capability to oil-soluble substances, thereby promoting thermal properties and chemical stability.

The water-insoluble substances are the substances that dissolve oil rather than water. Specific examples of the water-insoluble substances may include carotenoids, ceramide liquid crystals, oil-soluble vitamins, oil-soluble extracts, oil-soluble dyes, etc. In this regard, the carotenoids are at least one selected from the group consisting of astaxanthin, alpha-carotene, beta-carotene, zeaxanthin, lutein, lycopene, capsanthin, beta-cryptoxanthin, and canthaxanthin. The ceramide liquid crystals consist of ceramide, cholesterol, stearic acid, caprylic/capric triglyceride, and lecithin. The oil-soluble vitamins are at least one selected from the group consisting of vitamin A, vitamin D, vitamin E, vitamin K, and ubiquinone. The oil-soluble extracts are at least one selected from the group consisting of green tea, Chinese liquorice, Tokyo violet, rosemary, rosehip, evening primroses, centella, argan, avocado, olive, tamanu, wheat germ, sunflower, almond, macadamia, borage, calendula, tea tree, arnica, safflower, rapeseed flower, apricot seed, grape seed, soy oil, and camellia oil. The oil-soluble dyes are preferably at least one selected from naturally extracted oil-soluble dyes or synthetic oil-soluble dyes.

In order to minimize the loss of the water-insoluble substances possibly occurring during the preparation process prior to making the water-insoluble substances into the final stable form, it is preferable to dissolve the water-insoluble substances in a first solvent having a low boiling temperature in relation to water, such as methyl alcohol, ethyl alcohol, isopropyl alcohol, etc. and then add a stabilizer. The water-insoluble substances and the stabilizer are immobilized with the hollow silica in the presence of the first solvent having a relatively low boiling temperature. In this regard, using the solvent with the lower melting temperature can more minimize the damage of the water-insoluble substances by the heat.

The stabilizer consists of a compound having the property to dissolve in oil and containing a double bond or at least one molecular structure in which double bonds form a resonance structure. Specific examples of the stabilizer may include tocopherol or its derivatives or its salts, propyl gallate, lutein, vitamin C derivatives, gamma oryzanol, butylhydroxytoluene, butylhydroxyanisole, sesamole or its derivatives, cholesterol or its derivatives, lecithin or its derivatives, magnolia extract, green tea extract, etc.

The hollow silica in which the water-insoluble substance and the stabilizer are immobilized is the substance excellent in oil absorption and thermal stability. The oil absorption of the hollow silica used in the present invention is in the range of 80 ml/100g to 300 ml/100g. More preferably, the hollow silica as used herein has an oil absorption of 300 ml/100g. This can relatively raise the inclusion capability for the water-insoluble substance included in the final capsule and minimize the used amount of the hollow silica, thus offering a larger variety of choice in determining the content of the capsule wall.

The preferred inclusion method as used in the present invention includes the following steps.

In the preparation of the single-layered capsule, the water-insoluble substance, the stabilizer and the hollow silica are used at a weight ratio of 10 : 0.1 to 1 : 2 to 20.

Most of all, the water-insoluble substance and the stabilizer are completely dissolved in a first solvent under agitation, and the hollow silica is then added to form a first homogeneously dispersed solution. In order to remove the first solution of the solvent, a rotary evaporator is used to eliminate the solvent at appropriate temperature, pressure and rotational speed. After removal of the solvent, the remaining powder containing immobilized water-insoluble substance is measured in regards to the amount of the residual solvent using the high performance liquid chromatography analyzer. In detection of the residual solvent, the same preparation process is continued to eliminate the residual solvent. When no residual solvent is detected, the powder is collected.

(2) Preparation of Double-Layered Capsule

In the preparation of the double-layered capsule, the single-layered capsule and the hydrogel polymer blend are used at an amount of 10 to 80 wt.% and 20 to 90 wt.%, respectively, with respect to the total solid weight of the double-layered capsule.

The double-layered capsule uses the single-layered capsule as a core and the hydrogel polymer blend in the form of a shell. In other words, the single-layered capsule is homogeneously dispersed in a second solvent in which the hydrogel polymer blend is dissolved, and a drying is performed to prepare the double-layered capsule. The preferred method is using the spray drying process. In this regard, the double-layered capsule varies in the properties and forms depending on the type and proportion of the hydrogel polymer blend, the core-to-shell ratio, the solid content, and the spray drying temperature. It is therefore necessary to prepare the double-layered capsule in consideration of the above-mentioned factors in order to secure the required properties of the capsule. In the double-layered capsule thus formed, the hydrogel polymer blend prevents the flow of the water-soluble substances in and out of the capsule to the maximum according to the osmosis principle, whereas the gel phase immobilizing the water-soluble substances blocks the oil-soluble substances.

The hydrogel polymer blend includes at least two of hyaluronic acid, gellan gum, starch and starch derivatives, sodium alginate, agar, pectin, karageenan, locust bean gum, xanthan gum, guar gum, and water-soluble cellulose derivatives, which may be mixed together at an appropriate mixing ratio. The factors taken into consideration in this regard are the induced viscosity, swelling capacity, resistance to water, and skin feel. More specifically, the lower viscosity is preferred, and the swelling capacity is desirably 1.2 to 1.5 time in volume with respect to water when 1 % of the hydrogel polymer blend is added to water. When the swelling capacity is less than 1.2 time, the water absorption is relatively low to cause a considerable deterioration in the efficiency of the osmosis, which leads to the difficulty of realizing the defense mechanism against the external environments as required in the present invention. Contrarily, when the swelling capacity is greater than 1.5 time, the hydrogel polymer blend layer can be undesirably swollen to cause damages on the capsule after the preparation of the three-layered capsule.

As for the resistance to water, the standards appropriate to the resistance test as designed in the present invention is considered as the reference. More specifically, 50 g of water and 5 g of the capsule are added into a 100ml beaker and agitated for 24 hours with an agitator impeller operated at a constant speed. Six specimens before and after the fourth hour of agitation (that is, twelve specimens in total) are collected to compare the particle size distribution using a micro-scaled particle size analyzer. With low resistance to water, the proportion of fine powder in the particle size distribution tends to increase, leading to an abrupt drop of the average particle size. It can be therefore considered that the resistance to water is good if the average particle size of the capsule before the test is similar to that after the test. The desirable reference for this determination is adjusted by the combination and content of the hydrogel polymers and confined to within $\pm 5$ $\mu$m of the average particle diameter.

The skin feel is evaluated according to the sensory test and considered as "good" when it feels like there is no foreign object on the skin to which the hydrogel polymer blend capsule is applied after aged with water.

The desirable core-to-shell ratio is such that the core proportion is 50 to 80 wt.% whereas the shell proportion is 20 to 50 wt.%. Preferably, the core proportion is 70 to 80 wt.% whereas the shell proportion is 20 to 30 wt.%. Relatively, when the core proportion is less than 10 wt.%, the content of the water-insoluble substances is extremely low to deteriorate the effectiveness of the water-insoluble substances. When the core proportion is greater than 80 wt.%, the content of the capsule wall is too low to secure properties good enough to construct the double-layered capsule.

On the other hand, the solid content of the core and shell increases with the lower content of water or hydrous ethanol used as the second solvent. The solid content of the core and shell is appropriately 10 to 50 wt.%, preferably 15 to 35 wt.%. When the solid content of the core and shell is less than 10 wt.% or greater than 50 wt.%, the viscosity is too low or high to control the size of the capsule. Such a difficulty leads to the formation of minute particles or macro-particles and hence undesired capsules, consequently with deterioration in the yield of the desired product.

The spray drying temperature has a profound effect on the moisture content in the capsule and the shape of the capsule. Particularly, the more specific temperature interval exists when using the hydrogel polymer blend rather than general polymers as a wall material. The internal temperature of the spry dryer is suitably in the range of 85 to 115 °C, more preferably 95 to 105 °C. When the drying temperature is below 85 °C, the capsule is dried insufficiently and stuck to the inner wall of the spray dryer, making a loss to reduce the yield and producing the capsule with too high water content to secure appropriate properties. Contrarily, when the drying temperature is above 115 °C, it causes a deterioration in the stability of the water-soluble substance which is inherently poor in thermal stability, and the abrupt evaporation of water results in damages on the outer wall of the capsule, adversely affecting the shape of the capsule.

(3) Preparation of Three-Layered Capsule

In the preparation of the three-layered capsule, the double-layered capsule and the hydrophobic polymer are used in an amount of 50 to 90 wt.% and 10 to 50 wt.%, respectively, with respect to the total solid weight of the three-layered capsule.

The three-layered capsule can be prepared by homogeneously dispersing the capsule formed in the preparation step for the double-layered capsule in a third solvent containing a dissolved hydrophobic polymer and then performing a drying; or by dispersing functional pigments in a third solvent containing a dissolved hydrophobic polymer, adding the double-layered capsule to form a homogeneous dispersion, and then performing a drying. The preparation process for the three-layered capsule is appropriately the spry drying process or the fluidized bed coating process, most preferably the fluidized bed coating process that is available to form a uniform capsule layer. The three-layered capsule thus obtained contains the hydrophobic polymer formed into a support layer and used to prevent an abrupt entry of the water-soluble substances into the capsule. Also, the three-layered capsule further contains functional pigments to complete a capsule having a firm structure like a brick structure and a concealing function.

The three-layered capsule needs to be designed so as not to expose the double-layered capsule to the outermost layer. It is the properly selected hydrophobic polymer material that is in charge of offering stability to the capsule. Preferably, the three-layered capsule additionally includes functional pigments. The suitable hydrophobic polymer material as used herein includes at least one selected from zein, water-insoluble cellulose derivatives, a copolymer of quaternary ammonium acrylate and methacrylate, and shellac. The functional pigment as used herein includes at least one selected from titanium dioxide, mica, synthetic mica, boron nitride, talc, iron oxide, tar dye, lake pigment, and pearl. The third solvent as used herein is ethyl alcohol or hydrous ethanol. The functional pigment as used herein is at least one selected from the group consisting of titanium dioxide, mica, synthetic mica, boron nitride, talc, iron oxide, tar dye, lake pigment, pearl, etc.

In the case of using the functional pigment, the double-layered capsule, the hydrophobic polymer, and the functional pigment are used in an amount of 10 to 60 wt.%, 10 to 50 wt.%, and 10 to 80 wt.%, respectively, with respect to the total solid weight of the three-layered capsule.

The solution thus formed is sprayed on the double-layered capsule provided in the fluidized bed coating machine to prepare a three-layered capsule.

In this regard, the factors of greater importance in making the capsule coating uniform are the viscosity and adhesion of the solution, the feeding speed of the solution, and the drying temperature. The viscosity of the solution is determined by the proportions of the hydrophobic polymer and the third solvent. The suitable viscosity range is 10 cP to 300 cP, more preferably 50 cP to 180 cP. The adhesion of the solution is determined by the properties and concentration of the hydrophobic polymer material and the added amount of the functional pigment. The adhesive capacity is evaluated according to the sensory test to secure suitable conditions. The feeding speed of the solution and the drying temperature are determined by the speed of the feeding pump and the feeding temperature and internal pressure conditions of the fluidized bed coating machine. The conditions of the preparation process may vary depending on the type and state of the solution.

The three-layered capsule obtained by the preparation process may come in different particle diameters. More specifically, the average particle diameter is in the range of 50 $\mu$m to 3,000 $\mu$m. The above-mentioned conditions may vary depending on the particle diameter of the individual capsules.

Hereinafter, the present invention will be described with reference to Examples and Experimental Examples, which are not intended to limit the scope of the present invention.

<Comparative Examples 1 and 2 and Examples 1 to 5>

The individual ingredients of Table 1 are used to prepare a single-layered capsule of Comparative Example 1, a double-layered capsule of Comparative Example 2, and three-layered capsules of Examples 1 to 5 according to the following preparation method. In this regard, the content of each ingredient is indicated in terms of the weight.

[Table 1]

| Step | Component | Ingredient | Comparative Example | |
|------|-----------|------------|:---:|:---:|
| | | | 1 | 2 |
| 1 | Water-insoluble substance | Astaxanthin | 10 | 10 |
| | | Ceramide | – | – |
| | | Cholesterol | – | – |
| | | Stearic acid | – | – |
| | | Caprylic/capric triglyceride | – | – |
| | | Lecithin | – | – |
| | | Ubiquinone | – | – |

| Step | Component | Ingredient | | |
|---|---|---|---|---|
| | | Green tea extract | – | – |
| | | Red No. 223 | – | – |
| | Stabilizer | Tocopherol | 0.9 | 0.9 |
| | | Propyl gallate | 0.1 | 0.1 |
| | Hollow silica | SILNOS® 350 | 20 | 20 |
| | First solvent | Ethyl alcohol | 60 | 60 |
| 2 | Hydrogel polymer | Gellan gum | – | 10 |
| | | Starch | – | 20 |
| | Second solvent | Purified water | – | 150 |
| 3 | Hydrophobic polymer | Zein | – | – |
| | Functional pigment | Titanium dioxide | – | – |
| | Third solvent | Ethanol | – | – |
| | | Purified water | – | – |

| Step | Component | Ingredient | Example | | | | |
|---|---|---|---|---|---|---|---|
| | | | 1 | 2 | 3 | 4 | 5 |
| 1 | Water-insoluble substance | Astaxanthin | 10 | – | – | – | – |
| | | Ceramide | – | 5 | – | – | – |
| | | Cholesterol | – | 0.5 | – | – | – |
| | | Stearic acid | – | 1.5 | – | – | – |
| | | Caprylic/capric triglyceride | – | 3.5 | – | – | – |
| | | Lecithin | – | 0.5 | – | – | – |
| | | Ubiquinone | | | 10 | – | – |
| | | Green tea extract | – | – | – | 10 | – |
| | | Red No. 223 | – | – | – | – | 10 |
| | Stabilizer | Tocopherol | 0.9 | – | 1 | 0.7 | 1 |
| | | Propyl gallate | 0.1 | – | – | 0.3 | – |
| | Hollow silica | SILNOS® 350 | 20 | 20 | 20 | 20 | 20 |
| | First solvent | Ethyl alcohol | 60 | 60 | 60 | 60 | 60 |
| 2 | Hydrogel polymer | Gellan gum | 10 | 10 | 10 | 10 | 10 |
| | | Starch | 20 | 20 | 20 | 20 | 20 |
| | Second | Purified water | 150 | 150 | 150 | 150 | 150 |

| | | | | | | | | |
|---|---|---|---|---|---|---|---|---|
| | | solvent | | | | | | |
| 3 | Hydrophobic polymer | Zein | 9 | 9 | 9 | 9 | 9 |
| | Functional pigment | Titanium dioxide | 30 | 30 | 30 | 30 | 30 |
| | Third solvent | Ethanol | 200 | 200 | 200 | 200 | 200 |
| | | Purified water | 50 | 50 | 50 | 50 | 50 |

<Preparation Method>

(1) In the first step of the preparation process of Table 1, the water-insoluble substances and the stabilizers are dissolved in a first solvent at 20 °C. In this regard, the dissolution in the first solvent is performed at 50 to 60 °C only in Example 2. The hollow silica is added, and the resultant solution is stirred uniformly with a mechanical agitator at 500 to 1,000 rpm for 30 minutes.

(2) The dispersed solution prepared in step (1) is removed of the first solvent through evaporation on a rotary evaporator to yield a single-layered capsule powder. As for the conditions of the process, the temperature is 20 to 30 °C; the pressure is 5 to 6 bar; and the rotational speed is 50 to 100 rpm.

(3) In the second step of the preparation process, the hydrogel polymers are dissolved in the second solvent. As for the order of preparation, in order to facilitate dissolution of the starch, the second solvent is warmed up to 90 to 100 °C that is the gelatinization temperature and then cooled down to 20 to 30 °C. Gellan gum is then dissolved in the second solvent.

(4) To the solvent prepared in the step (3) is added the powder prepared in the step (2). The solution is then uniformly dispersed with a mechanic agitator at a speed of 1,000 to 1,500 rpm for 30 minutes.

(5) The dispersed solution thus prepared in the step (4) is removed of the second solvent through evaporation with a spray dryer to yield a double-layered capsule powder. As for the conditions of the process, the outlet temperature of the spray dryer is 80 to 100 °C; the added amount of the dispersion is 30 to 150 ml/min; and the rotational speed of the atomizer is 6,500 to 10,000 rpm.

(6) In the third step of the preparation process, the hydrophobic polymers are dissolved in the third solvent, and the functional pigments are added and dispersed. Zein used as the hydrophobic polymer is completely dissolved in hydrous ethanol used as a mixed solvent at dissolution temperature of 20 to 30 °C. Subsequently, titanium dioxide is added to the solution and uniformly dispersed with a mechanical agitator at a speed of 1,000 to 1,500 rpm for 30 minutes.

(7) The powder prepared in the step (5) is added to the dispersed solution of the step (6) and uniformly dispersed with a mechanical agitator at a speed of 500 to 1,000 rpm for 30 minutes.

(8) The dispersed solution prepared in the step (7) is removed of the third solvent through evaporation using a fluidized bed coating machine to yield a three-layered capsule powder.

As for the conditions of the process, the outlet temperature of the fluidized bed coating machine is maintained at 50 to 70 °C.

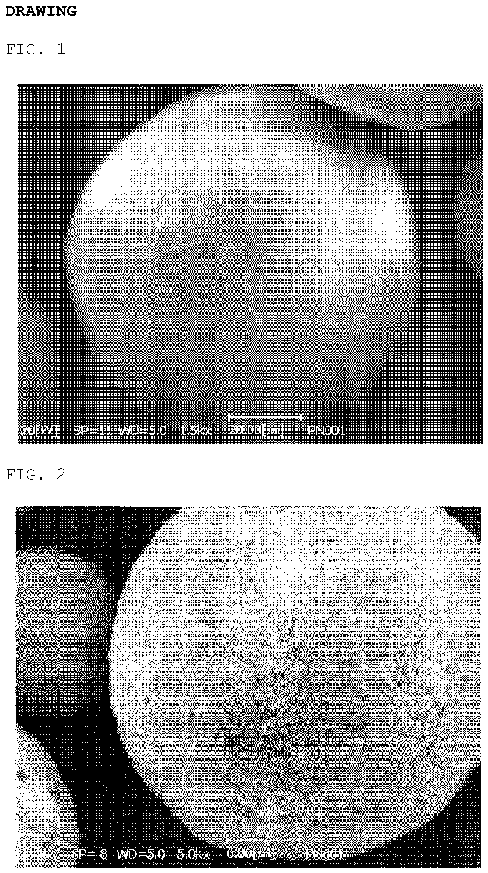

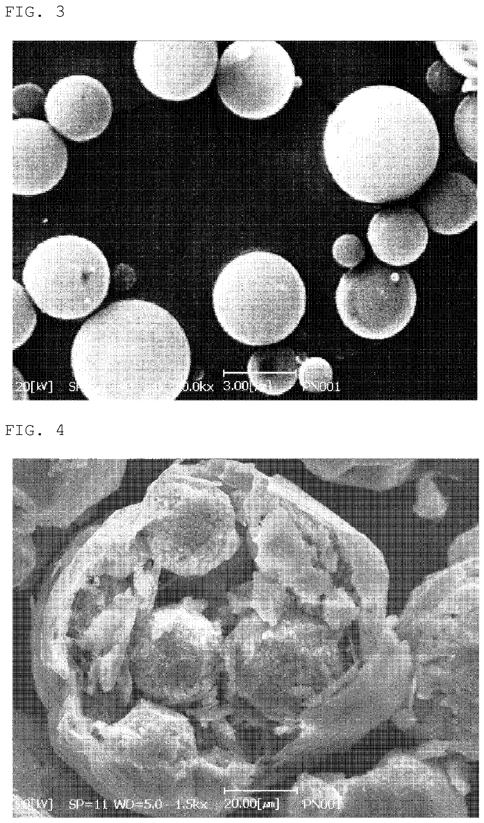

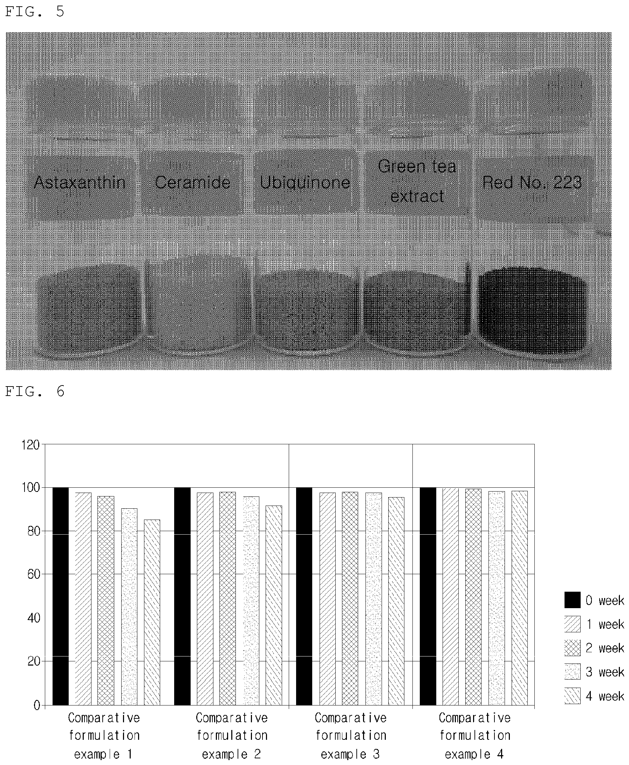

FIGS. 1, 2 and 3 are SEM images of the single-layered capsule, the double-layered capsule, and the three-layered capsule prepared in the first, second and third steps of the preparation process, respectively. FIG. 4 is an SEM image of the double-layered capsule with the outer wall of the capsule purposely damaged. FIG. 5 is an image showing the outer appearance of the three-layered capsules prepared in Examples 1 to 5.

As can be seen from FIGS. 1, 2 and 3, each of the single-layered capsule, the double-layered capsule, and the three-layered capsule as prepared in the first, second and third preparation steps of the present invention has a separated spherical form, with the outer capsule enclosing the inner capsule. It is shown that a number of double-layered capsules are embedded in the three-layered capsule of FIG. 4.

<Comparative Formulation Examples 1, 2 and 3 and Formulation Examples 1 to 5>

The capsules prepared in Comparative Examples and Examples using the ingredients of Table 2 are added at the same amount of the water-insoluble substances to prepare formulations. The individual formulations are evaluated in regards to the change in the content of each ingredient under the defined temperature and sunlight conditions over time. The evaluation results are presented in Table 3. The content of each ingredient is indicated in terms of wt.%.

[Table 2]

| Ingredient | Comparative Formulation Example | | |
|---|---|---|---|
| | 1 | 2 | 3 |
| Stearyl alcohol | 1.5 | 1.5 | 1.5 |
| Glyceryl stearate | 1.5 | 1.5 | 1.5 |
| Glyceryl stearate/ PEG 100 | 1.5 | 1.5 | 1.5 |
| Polysorbate 60 | 1.0 | 1.0 | 1.0 |
| Sorbitan sesquioleate | 0.5 | 0.5 | 0.5 |
| Vegetable squalene | 3.0 | 3.0 | 3.0 |
| Hydrogenated polydecene | 3.0 | 3.0 | 3.0 |
| Dimethicone | 0.5 | 0.5 | 0.5 |
| Butylene glycol | 5.0 | 5.0 | 5.0 |
| Astaxanthin | 0.1 | - | - |
| Capsule of Comparative Example 1 | - | 0.5 | - |
| Capsule of Comparative Example 2 | - | - | 1.0 |
| Capsule of Example 1 | - | - | - |
| Capsule of Example 2 | - | - | - |
| Capsule of Example 3 | - | - | - |
| Capsule of Example 4 | - | - | - |
| Capsule of Example 5 | - | - | - |
| Preservative | 0.7 | 0.7 | 0.7 |
| Fragrance | 0.1 | 0.1 | 0.1 |
| Purified water | To 100 | To 100 | To 100 |

| Ingredient | Formulation Example | | | | |
|---|---|---|---|---|---|
| | 1 | 2 | 3 | 4 | 5 |
| Stearyl alcohol | 1.5 | 1.5 | 1.5 | 1.5 | 1.5 |
| Glyceryl stearate | 1.5 | 1.5 | 1.5 | 1.5 | 1.5 |
| Glyceryl stearate/ PEG 100 | 1.5 | 1.5 | 1.5 | 1.5 | 1.5 |
| Polysorbate 60 | 1.0 | 1.0 | 1.0 | 1.0 | 1.0 |
| Sorbitan sesquioleate | 0.5 | 0.5 | 0.5 | 0.5 | 0.5 |
| Vegetable squalene | 3.0 | 3.0 | 3.0 | 3.0 | 3.0 |
| Hydrogenated polydecene | 3.0 | 3.0 | 3.0 | 3.0 | 3.0 |
| Dimethicone | 0.5 | 0.5 | 0.5 | 0.5 | 0.5 |
| Butylene glycol | 5.0 | 5.0 | 5.0 | 5.0 | 5.0 |
| Astaxanthin | - | - | - | - | - |
| Capsule of Comparative Example 1 | - | - | - | - | - |
| Capsule of Comparative Example 2 | - | - | - | - | - |
| Capsule of Example 1 | 2.0 | - | - | - | - |

(continued)

| Ingredient | Formulation Example | | | | |
|---|---|---|---|---|---|
| | 1 | 2 | 3 | 4 | 5 |
| Capsule of Example 2 | - | 2.0 | - | - | - |
| Capsule of Example 3 | - | - | 2.0 | - | - |
| Capsule of Example 4 | - | - | - | 2.0 | - |
| Capsule of Example 5 | - | - | - | - | 2.0 |
| Preservative | 0.7 | 0.7 | 0.7 | 0.7 | 0.7 |
| Fragrance | 0.1 | 0.1 | 0.1 | 0.1 | 0.1 |
| Purified water | To 100 | To 100 | To 100 | To 100 | To 100 |

[Table 3]

| Time | Environment conditions | Comparative Formulation Example (%) | | |
|---|---|---|---|---|
| | | 1 | 2 | 3 |
| 0 week | – | 99.6 | 99.5 | 99.8 |
| 1 week | 4 °C | 97.3 | 97.8 | 97.5 |
| | 25 °C | 95.2 | 96.9 | 98.6 |
| | 50 °C | 52.2 | 82.1 | 83.6 |
| | Exposed to sunlight | 95.5 | 97.9 | 97.7 |
| 2 weeks | 4 °C | 95.5 | 97.6 | 97.1 |
| | 25 °C | 90.4 | 92.5 | 96.5 |
| | 50 °C | 25.7 | 75.2 | 80.2 |
| | Exposed to sunlight | 92.9 | 95.8 | 95.7 |

| | | | | |
|---|---|---|---|---|
| 3 weeks | 4 °C | 89.1 | 95.3 | 97.0 |
| | 25 °C | 85.9 | 91.0 | 94.4 |
| | 50 °C | 2.5 | 69.4 | 77.5 |
| | Exposed to sunlight | 86.4 | 95.2 | 94.3 |
| 4 weeks | 4 °C | 85.0 | 91.3 | 95.3 |
| | 25 °C | 78.3 | 85.4 | 90.2 |
| | 50 °C | 0.0 | 57.8 | 76.8 |
| | Exposed to sunlight | 78.2 | 88.5 | 91.5 |

| Time | Environment conditions | Formulation Example (%) | | | |
|---|---|---|---|---|---|
| | | 1 | 2 | 3 | 4 |
| 0 week | – | 99.8 | 100.0 | 100.0 | 99.7 |
| 1 week | 4 °C | 99.3 | 99.3 | 99.8 | 99.6 |
| | 25 °C | 97.9 | 99.7 | 99.4 | 99.5 |
| | 50 °C | 97.4 | 99.8 | 99.7 | 99.4 |
| | Exposed to sunlight | 98.1 | 99.7 | 98.9 | 99.0 |
| 2 weeks | 4 °C | 99.0 | 99.3 | 100.0 | 99.6 |
| | 25 °C | 98.0 | 99.8 | 99.8 | 99.8 |
| | 50 °C | 95.1 | 98.5 | 99.6 | 99.0 |
| | Exposed to sunlight | 97.2 | 99.8 | 98.2 | 99.0 |
| 3 weeks | 4 °C | 98.1 | 100.0 | 99.5 | 99.5 |
| | 25 °C | 97.3 | 99.6 | 99.5 | 98.4 |
| | 50 °C | 92.6 | 100.0 | 96.4 | 97.7 |
| | Exposed to sunlight | 96.8 | 99.9 | 96.5 | 95.0 |
| 4 weeks | 4 °C | 98.2 | 99.4 | 99.3 | 98.8 |
| | 25 °C | 96.9 | 98.8 | 98.8 | 98.0 |
| | 50 °C | 92.7 | 99.7 | 95.3 | 96.7 |
| | Exposed to sunlight | 94.4 | 99.5 | 96.1 | 96.2 |

<Experimental Example 1> Analysis Test of Astaxanthin Content

1. Analysis conditions and method

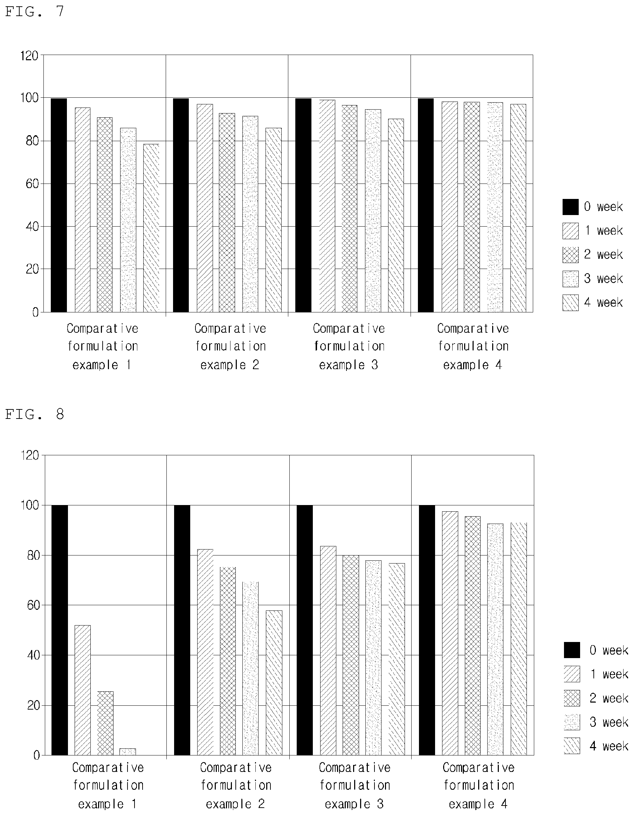

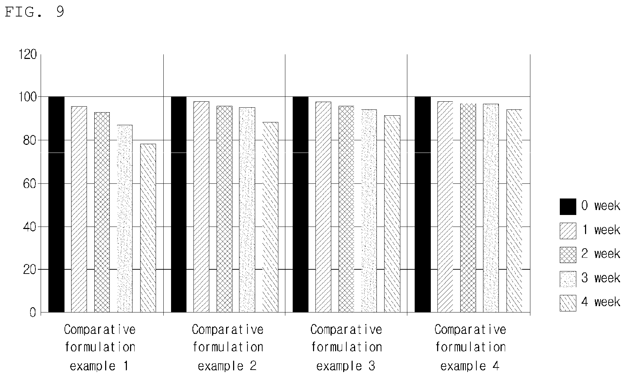

The capsules of Comparative Examples 1 and 2 and the capsule of Example 1 in Table 1 are measured in regards to activity according to an analysis test using the astaxanthin content of 5 % as a control under the following HPLC analysis conditions. The changes in the temperature stability (at 4 °C, 25 °C and 50 °C) and the sunlight stability are presented in the graphs of FIGS. 6 to 9, respectively.

HPLC column: Zorbax Eclipse XDB-C18 (2.1 x 150 mm, 5 μm) Temperature: 30 °C
Mobile phase: methyl tertiary butyl ether : methanol = 15 : 85 to 80 : 20
Flux: 0.12 ml/min
Detection wavelength: UV 475 nm

(1) Put 0.1 g of the control or the defined capsule specimen in a 10ml volumetric flask and add 10 ml of chloroform.
(2) Ultrasonicate with an ultrasonicator for 30 minutes.
(3) Shaken with a shaking apparatus, filtered with a 4.5μm filter and then analyze.

2. Analysis results

As shown in FIGS. 6 to 9, the comparative formulation examples 1, 2 and 3 are deteriorated in stability over time, and the formulation example 1 prepared according to the present invention has a constant stability.

Even the non-encapsulated materials are relatively good in the stability at a relatively low temperature out of the temperature interval, whereas the stability is abruptly deteriorated at higher temperature. This phenomenon improves as the progress of the encapsulation by the steps, and the improvement is maximized in the case of the three-layered capsule.

It can be seen from the experimental results that the high stability is maintained when astaxanthin is encapsulated into a three-layered structure and applied to the formulation.

<Experimental Example 2> Analysis Test of Ceramide Content

1. Analysis conditions and method

In order to determine whether the capsule of Example 2 in Table 1 has a change in the content over time under different environments, the specimen of Formulation Example 2 is used in the evaluation test under the following HPLC analysis conditions.

HPLC column: ACE5-C18 (4.6 x 150 $\mu$m, 5 $\mu$m)
Guard columns: ACE5-C18 (4.6 x 12.5 $\mu$m, 5 $\mu$m)
Mobile phase: methanol 100 %
Feeding volume: 10 $\mu$l
Flux: 1.0 ml/min
Detection wavelength: UV 203 nm

(1) Put 0.1 g of the capsule specimen in a 10ml flask and add 10 ml of methanol.
(2) Shaken with a shaking apparatus for 30 minutes and analyze.

2. Analysis results

According to Table 3, the analysis on the specimen of Formulation Example 2 shows that there is almost no change in the content over time.

In addition, the formulation stability (phase separation or precipitation) is also maintained at high level. It is therefore expected that the formulation can be made easier by applying the ceramide liquid crystal capsule to the formulation.

<Experimental Example 3> Analysis Test of Ubiquinone Content

1. Analysis conditions and method

In order to determine whether the capsule of Example 3 in Table 1 has a change in the content over time under different environments, the specimen of Formulation Example 3 is used in the evaluation test under the following HPLC analysis conditions.

HPLC column: ACE5-C18 (4.6 x 150 $\mu$m, 5 $\mu$m)
Guard columns: ACE5-C18 (4.6 x 12.5 $\mu$m, 5 $\mu$m)
Mobile phase: methanol : ethanol = 13 : 7
Temperature: 35 °C
Feeding volume: 50 $\mu$l
Flux: 1.5 ml/min
Detection wavelength: UV 275 nm

(1) Put 0.1 g of the capsule specimen in a 10ml flask and add 10 ml of ethanol.
(2) Shaken with a shaking apparatus and ultrasonicate for 30 minutes to analyze.

2. Analysis results

According to Table 3, the analysis on the specimen of Formulation Example 3 shows that there is almost no change in the content over time.

As can be seen from the analysis results, it is possible to maintain high stability when a three-layered capsule prepared using ubiquinone is applied to the formulation.

<Experimental Example 4> Analysis Test of Green Tea Extract Content

1. Analysis conditions and method

In order to determine whether the capsule of Example 4 in Table 1 has a change in the content over time under different environments, epigallocatechin gallate contained in the green tea extract is used as an index to analyze the specimen of Formulation Example 4 under the following HPLC analysis conditions.

HPLC column: ACE5-C18 (2.1 x 150 $\mu$m, 5 $\mu$m)
Guard columns: ACE5-C18 (2.1 x 12.5 $\mu$m, 5 $\mu$m)
Mobile phase: acetonitrile : acetic acid : methanol : purified water = 150 : 5 : 20 : 862
Feeding volume: 1 $\mu$l
Flux: 0.2 ml/min
Detection wavelength: UV 280 nm

    (1) Put 0.1 g of the capsule specimen in a 10ml flask and add 10 ml of methanol.
    (2) Shaken with a shaking apparatus for 30 minutes.
    (3) Ultrasonicate with an ultrasonicator for 30 minutes and put 1 ml of the specimen in a 1.5ml tube.
    (4) Centrifuge with a centrifugal separator at 5,000 rpm for 20 minutes to perform an analysis.

2. Analysis results

According to Table 3, the analysis on the specimen of Formulation Example 4 shows that there is almost no change in the content over time.

As can be seen from the analysis results, it is possible to maintain high stability when a three-layered capsule prepared using the green tea extract is applied to the formulation.

<Experimental Example 5> Colorimetric Analysis Using Red No. 223

1. Analysis conditions and method

In order to determine whether the capsule of Example 5 in Table 1 has a change in the sunlight stability over time under different environments, a color-difference meter is used as follow to perform a test. The analytical results are presented in Table 4.

Analysis instrument: Spectrophotometer ColorQuest XE
Mode type: RSIN
Area view: 0.780 in
UV filter: Nominal

    (1) Put 1 g of the capsule specimen in a 5ml quartz cell, position the quartz cell at a cell fixture and perform an analysis.

The inherent colors of the specimen with respect to the analysis results are expressed as L*, a*, and b*, and the color difference between the specimens is expressed in terms of E.

E is calculated as the following equation:

$$\triangle E^* = \sqrt{(L_2^* - L_1^*)^2 + (a_2^* - a_1^*)^2 - (b_2^* - b_1^*)^2}$$

When the value of E* is 1 or less, it means that there is no color change.

2. Analysis results

According to Table 4, the analysis on the specimen of Formulation Example 5 shows that the variation of color difference over time under sunlight is 1 or less, which implies no change in the color difference.
As can be seen from the analysis results, it is possible to maintain high stability when a three-layered capsule prepared using Red No. 223 is applied to the formulation.

[Table 4]

| Time | Formulation Example 5 | | | |
|---|---|---|---|---|
| | L* | a* | b* | ΔE |
| 0 week | 40.36 | 11.84 | 3.22 | - |
| 1 week | 40.12 | 11.87 | 3.23 | 0.24 |
| 2 weeks | 40.09 | 11.75 | 3.24 | 0.29 |
| 3 weeks | 40.23 | 11.88 | 3.20 | 0.14 |
| 4 weeks | 39.89 | 11.76 | 3.18 | 0.48 |

**Claims**

1. A stable three-layered capsule using a water-insoluble substance, the stable three-layered capsule comprising:

   a single-layered capsule comprising a water-insoluble substance, a stabilizer, and a hollow silica;
   a double-layered capsule having a hydrogel polymer blend surrounding the outside of the single-layered capsule; and
   a three-layered capsule having a hydrophobic polymer surrounding the outside of the double-layered capsule.

2. The stable three-layered capsule using a water-insoluble substance as claimed in claim 1, wherein the water-insoluble substance comprises at least one selected from the group consisting of a carotenoid, a ceramide liquid crystal, an oil-soluble vitamin, an oil-soluble extract, and an oil-soluble dye.

3. The stable three-layered capsule using a water-insoluble substance as claimed in claim 2, wherein the carotenoid comprises at least one selected from the group consisting of astaxanthin, alpha-carotene, beta-carotene, zeaxanthin, lutein, lycopene, capsanthin, beta-cryptoxanthin, and canthaxanthin.

4. The stable three-layered capsule using a water-insoluble substance as claimed in claim 2, wherein the ceramide liquid crystal comprises ceramide, cholesterol, stearic acid, caprylic/capric triglyceride, and lecithin.

5. The stable three-layered capsule using a water-insoluble substance as claimed in claim 2, wherein the oil-soluble vitamin comprises at least one selected from the group consisting of vitamin A, vitamin D, vitamin E, vitamin K, and ubiquinone.

6. The stable three-layered capsule using a water-insoluble substance as claimed in claim 2, wherein the oil-soluble extract comprises at least one selected from the group consisting of green tea, Chinese liquorice, Tokyo violet, rosemary, rosehip, evening primroses, centella, argan, avocado, olive, tamanu, wheat germ, sunflower, almond, macadamia, borage, calendula, tea tree, arnica, safflower, rapeseed flower, apricot seed, grape seed, soy oil, and camellia oil.

7. The stable three-layered capsule using a water-insoluble substance as claimed in claim 2, wherein the oil-soluble dye comprises at least one selected from a naturally extracted oil-soluble dye or a synthetic oil-soluble dye.

8. The stable three-layered capsule using a water-insoluble substance as claimed in claim 1, wherein the stabilizer comprises at least one selected from compounds having at least one double bond.

9. The stable three-layered capsule using a water-insoluble substance as claimed in claim 1, wherein the hollow silica comprises at least one hollow silica having an oil absorption of 80 ml/100g to 300 ml/100g.

10. The stable three-layered capsule using a water-insoluble substance as claimed in claim 1, wherein the hydrogel polymer blend comprises at least one selected from the group consisting of hyaluronic acid, gellan gum, starch and a starch derivative, sodium alginate, agar, pectin, karageenan, locust bean gum, xanthan gum, guar gum, and water-soluble cellulose derivatives.

11. The stable three-layered capsule using a water-insoluble substance as claimed in claim 1, wherein the hydrophobic polymer comprises at least one selected from the group consisting of zein, a water-insoluble cellulose derivative, a copolymer of quaternary ammonium acrylate and methacrylate, and shellac.

12. The stable three-layered capsule using a water-insoluble substance as claimed in claim 1, wherein the three-layered capsule has an average particle size of 50 $\mu$m to 3,000 $\mu$m.

13. A method for preparing a stable three-layered capsule using a water-insoluble substance, the method comprising:

mixing a water-insoluble substance, a stabilizer, and a hollow silica with a first solvent to achieve a homogeneous inclusion and then performing a drying to prepare a single-layered capsule, wherein the first solvent comprises any one selected from the group consisting of methyl alcohol, ethyl alcohol, and isopropyl alcohol;
homogeneously dispersing the single-layered capsule in a second solvent selected from water or hydrous ethanol containing a dissolved hydrogel polymer blend and then performing a drying to prepare a double-layered capsule; and
homogeneously dispersing the double-layered capsule in a third solvent selected from ethyl alcohol or hydrous ethanol containing a dissolved hydrophobic polymer and then performing a drying to prepare a three-layered capsule; or dispersing a functional pigment having an additional concealing function in a third solvent containing a dissolved hydrophobic polymer, adding the double-layered capsule to achieve a homogeneously dispersion, and then performing a drying to prepare a three-layered capsule.

14. The method as claimed in claim 13, wherein the step of preparing a three-layered capsule comprises dispersing a functional pigment having a concealing function in a third solvent containing a dissolved hydrophobic polymer, adding the double-layered capsule to achieve a homogeneous dispersion, and then performing a drying.

15. The method as claimed in claim 14, wherein the functional pigment comprises at least one selected from the group consisting of titanium dioxide, mica, synthetic mica, boron nitride, talc, iron oxide, tar dye, lake pigment, and pearl.

16. The method as claimed in claim 13, wherein in the preparation of the single-layered capsule, the water-insoluble substance, the stabilizer and the hollow silica are used at a weight ratio of 1 to 10 : 0.1 to 1 : 2 to 20.

17. The method as claimed in claim 13, wherein in the preparation of the double-layered capsule, the single-layered capsule and the hydrogel polymer blend are used in an amount of 10 to 80 wt.% and 20 to 90 wt.%, respectively, with respect to the total solid weight of the double-layered capsule.

18. The method as claimed in claim 13, wherein in the preparation of the three-layered capsule, the double-layered capsule and the hydrophobic polymer are used in an amount of 50 to 90 wt.% and 10 to 50 wt.%, respectively, with respect to the total solid weight of the three-layered capsule.

19. The method as claimed in claim 14, wherein in the preparation of the three-layered capsule, the double-layered capsule, the hydrophobic polymer and the functional pigment are used in an amount of 10 to 60 wt.%, 10 to 50 wt.% and 10 to 80 wt.%, respectively, with respect to the total solid weight of the three-layered capsule.

20. A cosmetic composition comprising the three-layered capsule prepared according to claim 1.

**Patentansprüche**

1. Stabile dreilagige Kapsel, die eine wasserunlösliche Substanz verwendet, wobei die stabile dreilagige Kapsel umfasst:

eine einlagige Kapsel, die eine wasserunlösliche Substanz, einen Stabilisator und Hohlkern-Silika umfasst;

eine doppellagige Kapsel mit einer Hydrogelpolymermischung, die die Außenseite der einlagigen Kapsel umgibt; und

eine dreilagige Kapsel mit einem hydrophoben Polymer, das die Außenseite der doppellagigen Kapsel umgibt.

2. Stabile dreilagige Kapsel, die eine wasserunlösliche Substanz verwendet, nach Anspruch 1, wobei die wasserunlösliche Substanz mindestens eine umfasst ausgewählt aus der Gruppe bestehend aus einem Carotinoid, einem Ceramid-Flüssigkristall, einem öllöslichen Vitamin, einem öllöslichen Extrakt und einem öllöslichen Farbstoff.

3. Stabile dreilagige Kapsel, die eine wasserunlösliche Substanz verwendet, nach Anspruch 2, wobei das Carotinoid mindestens eines umfasst ausgewählt aus der Gruppe bestehend aus Astaxanthin, Alpha-Carotin, Beta-Carotin, Zeaxanthin, Lutein, Lycopin, Capsanthin, Beta-Cryptoxanthin und Canthaxanthin.

4. Stabile dreilagige Kapsel, die eine wasserunlösliche Substanz verwendet, nach Anspruch 2, wobei der Ceramid-Flüssigkristall Ceramid, Cholesterin, Stearinsäure, Caprylic/Capric Triglycerid und Lecithin umfasst.

5. Stabile dreilagige Kapsel, die eine wasserunlösliche Substanz verwendet, nach Anspruch 2, wobei das öllösliche Vitamin mindestens eines umfasst ausgewählt aus der Gruppe bestehend aus Vitamin A, Vitamin D, Vitamin E, Vitamin K und Ubichinon.

6. Stabile dreilagige Kapsel, die eine wasserunlösliche Substanz verwendet, nach Anspruch 2, wobei der öllösliche Extrakt mindestens einen umfasst ausgewählt aus der Gruppe bestehend aus grünem Tee, chinesischem Süßholz, Viola Herba, Rosmarin, Hagebutte, Nachtkerze, Centella, Argan, Avocado, Olive, Tamanu, Weizenkeim, Sonnenblume, Mandel, Macadamia, Borretsch, Ringelblume, Teebaum, Arnika, Färberdistel, Raps, Aprikosenkern, Traubenkern, Sojaöl und Kamelienöl.

7. Stabile dreilagige Kapsel, die eine wasserunlösliche Substanz verwendet, nach Anspruch 2, wobei der öllösliche Farbstoff mindestens einen umfasst ausgewählt aus einem natürlich extrahierten öllöslichen Farbstoff oder einem synthetischen öllöslichen Farbstoff.

8. Stabile dreilagige Kapsel, die eine wasserunlösliche Substanz verwendet, nach Anspruch 1, wobei der Stabilisator mindestens eine Verbindung umfasst ausgewählt aus Verbindungen mit mindestens einer Doppelbindung.

9. Stabile dreilagige Kapsel, die eine wasserunlösliche Substanz verwendet, nach Anspruch 1, wobei das Hohlkern-Silika mindestens ein Hohlkern-Silika mit einer Ölabsorption von 80 ml/100 g bis 300 ml/100 g umfasst.

10. Stabile dreilagige Kapsel, die eine wasserunlösliche Substanz verwendet, nach Anspruch 1, wobei die Hydrogelpolymermischung mindestens eines umfasst ausgewählt aus der Gruppe bestehend aus Hyaluronsäure, Gellan, Stärke und einem Stärkederivat, Natriumalginat, Agar, Pektin, Karageenan, Johannisbrotkernmehl, Xanthangummi, Guargummi und wasserlöslichen Cellulosederivaten.

11. Stabile dreilagige Kapsel, die eine wasserunlösliche Substanz verwendet, nach Anspruch 1, wobei das hydrophobe Polymer mindestens eines umfasst ausgewählt aus der Gruppe bestehend aus Zein, einem wasserunlöslichen Cellulosederivat, einem Copolymer aus quartärem Ammoniumacrylat und Methacrylat und Schellack.

12. Stabile dreilagige Kapsel, die eine wasserunlösliche Substanz verwendet, nach Anspruch 1, wobei die dreilagige Kapsel eine mittlere Teilchengröße von 50 $\mu$m bis 3000 $\mu$m aufweist.

13. Verfahren zur Herstellung einer stabilen dreilagigen Kapsel, die eine wasserunlösliche Substanz verwendet, wobei das Verfahren umfasst:

Mischen einer wasserunlöslichen Substanz, eines Stabilisators und Hohlkern-Silika mit einem ersten Lösungsmittel, um eine homogene Einlagerung zu erreichen, und dann Durchführen eines Trocknens, um eine einlagige Kapsel herzustellen, wobei das erste Lösungsmittel eines umfasst ausgewählt aus der Gruppe bestehend aus Methylalkohol, Ethylalkohol und Isopropylalkohol;

homogenes Dispergieren der einlagigen Kapsel in einem zweiten Lösungsmittel, ausgewählt aus Wasser oder wasserhaltigem Ethanol, das eine gelöste Hydrogelpolymermischung enthält, und dann Durchführen eines Trocknens zur Herstellung einer doppellagigen Kapsel; und

homogenes Dispergieren der doppellagigen Kapsel in einem dritten Lösungsmittel, ausgewählt aus Ethylalkohol oder wasserhaltigem Ethanol, das ein gelöstes hydrophobes Polymer enthält, und dann Durchführen eines Trocknens zur Herstellung einer dreilagigen Kapsel; oder Dispergieren eines funktionellen Pigments mit einer zusätzlichen abdeckenden Funktion in einem dritten Lösungsmittel, das ein gelöstes hydrophobes Polymer enthält, Zugeben der doppellagigen Kapsel, um eine homogene Dispersion zu erhalten, und dann Durchführen eines Trocknens, um eine dreilagige Kapsel herzustellen.

14. Verfahren nach Anspruch 13, wobei der Schritt des Herstellens einer dreilagigen Kapsel ein Dispergieren eines funktionellen Pigments mit einer abdeckenden Funktion in einem dritten Lösungsmittel, das ein gelöstes hydrophobes Polymer enthält, Zugeben der doppellagigen Kapsel zum Erzielen einer homogenen Dispersion und dann Durchführen einer Trocknung umfasst.

15. Verfahren nach Anspruch 14, wobei das funktionelle Pigment mindestens eines umfasst ausgewählt aus der Gruppe bestehend aus Titandioxid, Glimmer, synthetischem Glimmer, Bornitrid, Talk, Eisenoxid, Teerfarbstoff, Lake-Pigment und Perl.

16. Verfahren nach Anspruch 13, wobei bei der Herstellung der einlagigen Kapsel die wasserunlösliche Substanz, der Stabilisator und das Hohlkern-Silika in einem Gewichtsverhältnis von 1 bis 10 : 0,1 bis 1 : 2 bis 20 verwendet werden.

17. Verfahren nach Anspruch 13, wobei bei der Herstellung der doppellagigen Kapsel die einlagige Kapsel und die Hydrogelpolymermischung in einer Menge von 10 bis 80 Gew.-% bzw. 20 bis 90 Gew.-% verwendet werden, bezogen auf das Gesamtfeststoffgewicht der doppellagigen Kapsel.

18. Verfahren nach Anspruch 13, wobei bei der Herstellung der dreilagigen Kapsel die doppellagige Kapsel und das hydrophobe Polymer in einer Menge von 50 bis 90 Gew.-% bzw. 10 bis 50 Gew.-% verwendet werden, bezogen auf das Gesamtfeststoffgewicht der dreilagigen Kapsel.

19. Verfahren nach Anspruch 14, wobei bei der Herstellung der dreilagigen Kapsel die doppellagige Kapsel, das hydrophobe Polymer und das funktionelle Pigment in einer Menge von 10 bis 60 Gew.-%, 10 bis 50 Gew.-% bzw. 10 bis 80 Gew.-% verwendet werden, bezogen auf das Gesamtfeststoffgewicht der dreilagigen Kapsel.

20. Kosmetische Zusammensetzung umfassend die gemäß Anspruch 1 hergestellte dreilagige Kapsel.

## Revendications

1. Capsule tricouche stable utilisant une substance insoluble dans l'eau, ladite capsule tricouche stable comprenant :

   une capsule monocouche comprenant une substance insoluble dans l'eau, un stabilisant et une silice creuse ;
   une capsule bicouche ayant un mélange de polymère hydrogel entourant l'extérieur de la capsule monocouche ; et
   une capsule tricouche ayant un polymère hydrophobe entourant l'extérieur de la capsule bicouche.

2. Capsule tricouche stable utilisant une substance insoluble dans l'eau selon la revendication 1, dans laquelle la substance insoluble dans l'eau comprend au moins un élément choisi dans le groupe constitué d'un caroténoïde, un cristal liquide de céramide, une vitamine liposoluble, un extrait liposoluble et un colorant liposoluble.

3. Capsule tricouche stable utilisant une substance insoluble dans l'eau selon la revendication 2, dans laquelle le caroténoïde comprend au moins un élément choisi dans le groupe constitué d'astaxanthine, alpha-carotène, bêta-carotène, zéaxanthine, lutéine, lycopène, capsanthine, bêta-cryptoxanthine, et canthaxanthine.

4. Capsule tricouche stable utilisant une substance insoluble dans l'eau selon la revendication 2, dans laquelle le cristal liquide de céramide comprend du céramide, du cholestérol, de l'acide stéarique, du triglycéride caprylique/caprique et de la lécithine.

5. Capsule tricouche stable utilisant une substance insoluble dans l'eau selon la revendication 2, dans laquelle la vitamine liposoluble comprend au moins un élément choisi dans le groupe constitué de vitamine A, vitamine D, vitamine E, vitamine K et ubiquinone.

**6.** Capsule tricouche stable utilisant une substance insoluble dans l'eau selon la revendication 2, dans laquelle l'extrait liposoluble comprend au moins un élément choisi dans le groupe constitué du thé vert, réglisse chinoise, violette de Tokyo, romarin, cynorhodon, onagres, herbe du tigre, argan, avocat, olive, tamanu, germe de blé, tournesol, amande, noix de Macadémia, bourrache, souci, arbre à thé, arnica, carthame, fleur de ricin, noyau d'abricot, pépin de raisin, huile de soja, et huile de camélia.

**7.** Capsule tricouche stable utilisant une substance insoluble dans l'eau selon la revendication 2, dans laquelle le colorant liposoluble comprend au moins un élément choisi parmi un colorant liposoluble extrait naturellement ou un colorant liposoluble synthétique.

**8.** Capsule tricouche stable utilisant une substance insoluble dans l'eau selon la revendication 1, dans laquelle le stabilisant comprend au moins un élément choisi parmi les composés ayant au moins une double liaison.

**9.** Capsule tricouche stable utilisant une substance insoluble dans l'eau selon la revendication 1, dans laquelle la silice creuse comprend au moins une silice creuse ayant une absorption d'huile de 80 ml/100 g à 300 ml/100 g.

**10.** Capsule tricouche stable utilisant une substance insoluble dans l'eau selon la revendication 1, dans laquelle le mélange de polymère hydrogel comprend au moins un élément choisi dans le groupe constitué d'acide hyaluronique, gomme gellane, amidon et un dérivé d'amidon, alginate de sodium, agar, pectine, carraghénane, farine de graines de caroube, gomme xanthane, gomme guar, et dérivés de cellulose hydrosolubles.

**11.** Capsule tricouche stable utilisant une substance insoluble dans l'eau selon la revendication 1, dans laquelle le polymère hydrophobe comprend au moins un élément choisi dans le groupe constitué de zéine, un dérivé de cellulose insoluble dans l'eau, un copolymère d'acrylate et de méthacrylate d'ammonium quaternaire et gomme-laque.

**12.** Capsule tricouche stable utilisant une substance insoluble dans l'eau selon la revendication 1, dans laquelle la capsule tricouche a une taille de particules moyenne de 50 $\mu$m à 3000 $\mu$m.

**13.** Procédé de préparation d'une capsule tricouche stable utilisant une substance insoluble dans l'eau, ledit procédé comprenant :

le mélange d'une substance insoluble dans l'eau, d'un stabilisant et d'une silice creuse avec un premier solvant pour obtenir une inclusion homogène et puis la réalisation d'un séchage pour préparer une capsule monocouche, où le premier solvant comprend un élément quelconque choisi dans le groupe constitué d'alcool méthylique, alcool éthylique et alcool isopropylique ;
la dispersion homogène de la capsule monocouche dans un deuxième solvant choisi parmi l'eau ou l'éthanol aqueux contenant un mélange de polymère hydrogel dissous et puis la réalisation d'un séchage pour préparer une capsule bicouche ; et
la dispersion homogène de la capsule bicouche dans un troisième solvant choisi parmi l'alcool éthylique ou l'éthanol aqueux contenant un polymère hydrophobe dissous et puis la réalisation d'un séchage pour préparer une capsule tricouche ; ou la dispersion d'un pigment fonctionnel ayant une fonction additionnelle de masquage dans un troisième solvant contenant un polymère hydrophobe dissous, l'addition de la capsule bicouche afin d'obtenir une dispersion homogène et puis la réalisation d'un séchage pour préparer une capsule tricouche.

**14.** Procédé selon la revendication 13, dans lequel l'étape de préparation d'une capsule tricouche comprend la dispersion d'un pigment fonctionnel ayant une fonction de masquage dans un troisième solvant contenant un polymère hydrophobe dissous, l'addition de la capsule bicouche afin d'obtenir une dispersion homogène et ensuite la réalisation d'un séchage.

**15.** Procédé selon la revendication 14, dans lequel le pigment fonctionnel comprend au moins un élément choisi dans le groupe constitué de dioxyde de titane, mica, mica synthétique, nitrure de bore, talc, oxyde de fer, teinture de goudron, pigment-laque et nacre.

**16.** Procédé selon la revendication 13, dans lequel, dans la préparation de la capsule monocouche, la substance insoluble dans l'eau, le stabilisant et la silice creuse sont utilisés en un rapport en poids de 1 à 10 : 0,1 à 1 : 2 à 20.

**17.** Procédé selon la revendication 13, dans lequel, dans la préparation de la capsule bicouche, la capsule monocouche et le mélange de polymère hydrogel sont utilisés en une quantité de 10 à 80 % en poids et 20 à 90 % en poids, respectivement, par rapport au poids de matière solide total de la capsule bicouche.

18. Procédé selon la revendication 13, dans lequel, dans la préparation de la capsule tricouche, la capsule bicouche et le polymère hydrophobe sont utilisés en une quantité de 50 à 90 % en poids et 10 à 50 % en poids, respectivement, par rapport au poids de matière solide total de la capsule tricouche.

19. Procédé selon la revendication 14, dans lequel, dans la préparation de la capsule tricouche, la capsule bicouche, le polymère hydrophobe et le pigment fonctionnel sont utilisés en une quantité de 10 à 60 % en poids, 10 à 50 % en poids et 10 à 80 % en poids, respectivement, par rapport au poids de matière solide total de la capsule tricouche.

20. Composition cosmétique comprenant la capsule tricouche préparée selon la revendication 1.

**DRAWING**

FIG. 1

FIG. 2

FIG. 3

FIG. 4

FIG. 5

FIG. 6

FIG. 7

FIG. 8

FIG. 9

**EP 3 031 453 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020090070161